Europäisches Patentamt

European Patent Office

Office européen des brevets

(19)

(11) **EP 1 109 529 B1**

(12) **FASCICULE DE BREVET EUROPEEN**

(45) Date de publication et mention
de la délivrance du brevet:
**04.12.2002   Bulletin 2002/49**

(51) Int Cl.[7]: **A61K 7/48**, A61K 35/80

(21) Numéro de dépôt: **99941726.4**

(86) Numéro de dépôt international:
**PCT/FR99/02144**

(22) Date de dépôt: **09.09.1999**

(87) Numéro de publication internationale:
**WO 00/013660 (16.03.2000 Gazette 2000/11)**

(54) **UTILISATION D'UN EXTRAIT LIPIDIQUE DE L'ALGUE SKELETONEMA**

VERWENDUNG EINES LIPID-EXTRAKTS VON DER SKELETONEMA-ALGE

USE OF A LIPID EXTRACT OF THE SKELETONEMA ALGAE

(84) Etats contractants désignés:
**DE ES FR GB IT**

(30) Priorité: **09.09.1998  FR 9811241**

(43) Date de publication de la demande:
**27.06.2001   Bulletin 2001/26**

(73) Titulaire: **PARFUMS CHRISTIAN DIOR**
**75008 Paris (FR)**

(72) Inventeurs:
• **VIRON, Cécile**
**F-45000 Orléans (FR)**
• **KRZYCH, Valérie**
**F-45460 Les Bordes (FR)**
• **RENIMEL, Isabelle**
**F-45470 Trainou (FR)**

• **ANDRE, Patrice**
**F-45170 Neuville aux Bois (FR)**

(74) Mandataire: **Portal, Gérard**
**Cabinet Beau de Loménie**
**158, rue de l'Université**
**75340 Paris Cédex 07 (FR)**

(56) Documents cités:
**WO-A-94/24984**          **WO-A-94/28913**
**FR-A- 2 657 012**          **FR-A- 2 683 720**
**US-A- 5 767 095**

• **STN, Serveur de Bases de Données, Karlsruhe,
DE, Fichier Chemical Abstracts, Vol 112,
AN=6435 XP002125164**
• **STN, Serveur de Bases de Données, Karlsruhe,
DE, Fichier Chemical Abstracts, vol 121,
AN=77961 * résumé * XP002125163**

**Description**

L'invention concerne l'utilisation d'un extrait lipidique de l'algue Skeletonema dans le domaine cosmétique.

Cette algue est une algue du genre Skeletonema, en particulier l'algue Skeletonema tropicum, Skeletonema menzelii, Skeletonema potamos, Skeletonema subsalsum, Skeletonema pseudocostatum et Skeletonema costatum.

Elle concerne plus précisément des utilisations comme agent cosmétique d'un extrait lipidique de cette algue.

L'algue Skeletonema, en particulier l'algue Skeletonema tropicum, Skeletonema menzelii, Skeletonema potamos, Skeletonema subsalsum, Skeletonema peudocostatum et Skeletonema costatum (nommée SKC dans la suite du document), est une algue unicellulaire bien connue, du phylum des Chlorophytes, de l'embranchement des Chrysophycophytes, de la classe des Diatomophycées et de l'ordre des Centrales. Les Diatomophycées sont très répandues dans les eaux douces, marines, ou saumâtres. La vie des espèces de cette classe peut être planctonique ou benthique. Le protoplasme est enfermé dans un frustule siliceux. La plante davantage préférée est la plante Skeletonema costatum (SKC) est une espèce cosmopolite le plus souvent marine, que l'on trouve fréquemment associée aux efflorescences phytoplanctoniques des eaux côtières.

Les essais réalisés par les inventeurs ont permis de mettre en évidence une action enzymatique très surprenante des extraits lipidiques de cette algue. Les inventeurs ont mis en évidence une action d'inhibition sur la 3',5'-AMPc phosphodiestérase, ce qui a permis d'envisager son utilisation dans des produits cosmétiques.

La 3',5'-AMPc phosphodiestérase, désignée ci-après " phosphodiestérase " ou " PDE ", est l'enzyme qui transforme la 3',5'-adénosine monophosphate cyclique (AMPc), second messager qui intervient dans le contrôle du métabolisme cellulaire, en adénosine monophosphate (AMP), forme inactive dudit second messager. L'inhibition de la PDE par un agent inhibiteur permet en conséquence de maintenir un taux intracellulaire élevé d'AMPc, ce qui a pour effet notamment d'activer les protéines kinases A, et, par ce processus, permet en particulier de favoriser la dégradation des lipides.

De plus, il est également connu que l'AMPc intervient pour contrer certains processus inflammatoires (Front Matrix Biol., vol.6, pp. 193-205, Karger, Busel 1978, Skin inflammatory reactions and cyclic AMP, Georges Cehovic and Nguyen-Ba Giao). Il a également été décrit que la PDE augmente avec l'âge (S.K. Puri et L. Volicer, Mechanisms of Aging en Dev. (1981), 15, 239). De ce fait, l'inhibition de la PDE contribuera à retarder l'apparition des effets du vieillissement, en particulier au niveau cutané.

Ainsi, il a été mis en évidence par les inventeurs, que grâce à leur action inhibitrice sur la PDE, les extraits lipidiques de Skeletonema, en particulier des espèces précitées, et de préférence de SKC présentent un grand intérêt en cosmétique.

En effet, par la découverte de l'activité des extraits lipidiques de Skeletonema, en particulier des espèces précitées, et de préférence de SKC, inhibant l'action de la PDE, l'invention fournit différentes solutions dans le domaine de la cosmétique.

L'inhibition de la PDE conduit à une action amincissante, anti-inflammatoire, anti-cellulite et anti-vieillissement des compositions de l'invention.

Ainsi, selon un premier aspect, l'invention concerne l'utilisation d'un extrait lipidique de l'algue Skeletonema, obtenu par extraction dans un solvant organique, pour réaliser un soin cosmétique choisi parmi l'action amincissante, la réduction, l'élimination et la prévention des surcharges graisseuses sous-cutanées, notamment la cellulite.

Selon un deuxième aspect, l'invention concerne l'utilisation d'un extrait lipidique de l'algue Skeletonema, obtenu par extraction dans un solvant organique pour la fabrication d'une composition cosmétique destinée au soin de la peau pour inhiber la 3',5'-AMPc phosphodiestérase.

Selon une variante, l'algue Skeletonema est choisie dans le groupe consistant de S. tropicum, S. menzelii, S. potamos, S. subsalsum, S. pseudocostatum et S. costatum, en particulier un extrait lipidique total de ladite algue, obtenu par extraction dans un solvant organique.

Selon un mode de réalisation avantageux de l'invention, cet extrait lipidique est caractérisé en ce qu'il s'agit d'un extrait lipidique obtenu par extraction de l'algue Skeletonema dans un solvant organique, présentant un indice de polarité p' inférieur à environ 5,4, de préférence compris entre 2 et 4,5, et encore de préférence p' étant compris entre 4,2 et 4,4, et acceptable dans l'industrie cosmétique ou pharmaceutique. On se reportera, pour les indices de polarité des solvants, à l'article de L.R. SNYDER ; Classification of the solvent properties of common liquids ; Journal of Chromatography, 92 (1974), 223-230.

Selon encore un mode de réalisation avantageux de l'invention, l'extrait lipidique précité est obtenu par extraction de l'algue avec un solvant organique choisi parmi le groupe consistant de l'isopropanol, l'acétate d'éthyle, le dichlorométhane et le chloroforme.

Selon encore un autre mode de réalisation avantageux de l'invention, l'extrait précité est obtenu par extraction de l'algue avec de l'isopropanol.

A titre d'exemple, on peut rappeler les p' pour chaque solvant précité :

- isopropanol : 4,3
- acétate d'éthyle : 4,3
- chloroforme : 4,4
- dichlorométhane : 3,7.

Selon encore une variante de réalisation avantageuse, l'extraction est réalisée au reflux.

Selon encore une variante de réalisation avantageuse, l'algue est congelée avant d'être extraite avec le solvant, de préférence la congélation étant réalisée à une température située entre - 40°C et - 20°C environ et pendant une durée comprise de préférence entre 1 et 7 jours environ.

Selon encore une variante de réalisation avantageuse, l'algue congelée est plongée directement dans le solvant porté à température de reflux. Le choc thermique permet en effet de faciliter la décantation de la silice (issue du squelette des cellules des algues).

Selon encore une autre variante de réalisation avantageuse, avant toute autre opération d'extraction, on réalise une macération de l'algue dans le solvant organique à température ambiante et de préférence pendant une durée comprise entre 5 minutes et 80 minutes environ, et de préférence encore, pendant une durée comprise entre 20 minutes et 40 minutes environ.

Selon encore une autre variante de réalisation avantageuse, la quantité de solvant organique utilisée est comprise entre 0,1 litre et 20 litres environ de solvant, de préférence comprise entre 2 litres et 10 litres environ de solvant, pour 100 g d'algue, exprimés en poids sec d'algue.

Selon encore une autre variante de réalisation avantageuse, l'extraction peut-être réalisée sous atmosphère inerte, de préférence sous atmosphère saturée en azote. Ceci permet d'éviter en particulier une dégradation prononcée des molécules actives.

Le procédé peut comprendre également une simple macération (seule) à température ambiante et/ou un reflux ou un reflux seul, sous pression atmosphérique, de préférence sous atmosphère inerte, de préférence sous atmosphère saturée en azote.

La quantité de solvant organique utilisée est avantageusement comprise entre 0,1 litre et 20 litres environ de solvant, de préférence comprise entre 2 litres et 10 litres environ de solvant, pour 100 g d'algue, exprimés en poids sec d'algue.

L'extrait sera obtenu de préférence par une extraction en deux temps décrite ci-après. Une première étape d'extraction solide/liquide suivie d'une deuxième étape de fractionnement, de décoloration et de désodorisation.

L'extraction peut-être réalisée sous atmosphère inerte (saturée en azote), ce qui permet d'éviter en particulier une dégradation prononcée des molécules actives.

L'extrait est avantageusement obtenu par macération de l'algue dans un solvant, de préférence acceptable dans l'industrie cosmétique ou pharmaceutique et présente un indice de polarité p' inférieur à environ 5,4, de préférence compris entre 2 et 4,5 et encore de préférence p' étant compris entre 4,2 et 4,4.

L'extraction aura lieu de préférence à reflux.

Le temps de macération est de préférence compris entre environ 5 minutes et environ 80 minutes de préférence entre environ 20 minutes et environ 40 minutes.

L'ensemble (biomasse + solvant) est porté à reflux pendant une durée de environ 15 minutes à environ 2 heures, de préférence entre environ 20 minutes et environ 40 minutes, sous agitation (la température du solvant étant maintenue).

Après le refroidissement de la masse réactionnelle, l'extrait est filtré afin de réaliser la séparation biomasse épuisée / extrait lipidique dans le solvant.

L'extrait lipidique est ensuite concentré (Facteur de concentration égal à environ 71,5).

Pour commencer la seconde étape, l'extrait lipidique est repris dans le solvant à froid à raison de environ 5 kg à environ 15 kg de solvant pour 1 kg d'huile de préférence à raison de environ 9 kg à environ 11 kg de solvant pour 1 kg d'huile. L'agitation est prolongée pendant une durée comprise entre environ 10 minutes et environ 60 minutes de préférence entre environ 15 minutes et environ 25 minutes. Le jus est ensuite filtré, ce qui permet d'éliminer la boue collante résiduelle.

Le traitement de décoloration / désodorisation est ensuite effectué par exemple avec de la zéolithe et du charbon actif. Le taux de charbon peut être compris entre environ 1 % et environ 5 %, de préférence entre environ 1,5 % et environ 2 % et celui de zéolithe peut être compris entre environ 0,3% et environ 4 %, de préférence entre environ 0,8 % et environ 1,2 %. Le rapport charbon sur zéolithe peut être compris entre environ 1 et environ 4 de préférence entre environ 1,5 et environ 2.

La zéolithe et le charbon sont ensuite éliminés par exemple par filtration.

Des antioxydants peuvent être ajoutés pour la conservation des molécules actives tels que par exemple du DL alpha-tocophérol ou l'un de ses esters tel que le tocophérol phosphate, et/ou un ascorbate, en particulier un palmitate d'ascorbyle de préférence à une concentration comprise entre 0,001% et 5 % en poids par rapport au poids total dudit extrait. Ces antioxydants pourront être incorporés par une solution mère dans le solvant d'extraction.

Le filtrat et les antioxydants, qui ont pu être ajoutés, sont ensuite concentrés par exemple en batch jusqu'à l'obtention d'une huile de couleur brune.

Le conditionnement de cet extrait lipidique est réalisé de préférence sous gaz inerte tel que de l'azote afin de protéger les molécules actives.

La présente invention utilisee aussi un procédé de fabrication d'un extrait lipidique de l'algue Skeletonema, en particulier d'un extrait lipidique total de ladite algue, caractérisé en ce qu'on réalise une extraction de ladite algue avec un solvant organique pendant une période de temps suffisante pour réaliser ledit extrait organique lipidique. Les conditions d'extraction sont telles que définies dans l'exposé du premier aspect de l'invention. En particulier, l'extrait est avantageusement obtenu par extraction de l'algue Skeletonema dans un solvant organique présentant un indice de polarité p' inférieur à environ 5,4, de préférence compris entre 2 et 4,5, et encore de préférence p' étant compris entre 4,2 et 4,4, et acceptable dans l'industrie cosmétique ou pharmaceutique.

Diverses variantes de réalisation du procédé de fabrication apparaissent clairement à l'homme de l'art à partir de la description précédente relativement à la définition de l'extrait organique lipidique dans le cadre du premier aspect.

L'invention est mise en oeuvre dans le cadre de la fabrication d'une composition cosmétique, caractérisée en ce qu'elle comprend à titre de principe actif, un extrait lipidique précité de l'algue Skeletonema précitée, en particulier un extrait lipidique total de ladite algue, en présence d'un véhicule cosmétiquement acceptable.

Par ailleurs, comme précédemment indiqué, l'algue Skeletonema est en particulier choisie parmi le groupe consistant de l'algue Skeletonema tropicum, Skeletonema menzelii, Skeletonema potamos, Skeletonema subsalsum, Skeletonema pseudocostatum et Skeletonema costatum.

Selon un mode de réalisation avantageux, cette composition est caractérisée en ce qu'il s'agit d'une composition cosmétique destinée au soin de la peau, contenant une quantité cosmétiquement efficace de l'extrait lipidique de l'algue Skeletonema précitée pour lutter contre les effets du vieillissement cutané.

Selon encore un mode de réalisation avantageux, cette composition est caractérisée en ce qu'il s'agit d'une composition cosmétique destinée au soin de la peau, contenant une quantité cosmétiquement efficace de l'extrait lipidique de l'algue Skeletonema précitée pour obtenir une action amincissante.

Selon encore un autre mode de réalisation avantageux de l'invention, cette composition est caractérisée en ce qu'il s'agit d'une composition cosmétique destinée au soin de la peau, contenant une quantité cosmétiquement efficace de l'extrait lipidique de l'algue Skeletonema précitée pour obtenir une action anti-cellulite.

Les compositions selon l'invention peuvent être formulées selon toute forme acceptable pour leur emploi en cosmétologie. Il peut, en particulier, s'agir d'une composition sous forme appropriée pour une application topique, en particulier sous forme d'une crème ou d'un gel, en particulier d'une crème ou d'un gel pour le visage, les mains, le buste ou le corps.

Selon encore un autre mode de réalisation avantageux, cette composition comprend de 0,001 % à 10 % environ, et en particulier de 0,02 % à 1 % environ en poids sec dudit extrait lipidique par rapport au poids total de la composition finale.

D'autres caractéristiques particulières de cette composition cosmétique, notamment de l'extrait, résultent de la description précédente et suivante.

Selon encore un autre mode de réalisation avantageux, cette composition comprend aussi au moins un agent antioxydant, en particulier le DL α-tocophérol, ou l'un de ses sels ou esters tel que le tocophérolphosphate, et/ou un ascorbate, en particulier le palmitate d'ascorbyle, de préférence à une concentration comprise entre 0,001 % et 5 % environ en poids, par rapport au poids total dudit extrait.

Selon un troisième aspect, l'invention concerne l'utilisation d'un extrait lipidique de l'algue Skeletonema en particulier d'un extrait lipidique total de ladite algue, comme agent cosmétique, en présence d'un véhicule cosmétiquement acceptable, pour la fabrication d'une composition cosmétique.

Cet agent cosmétique sera notamment utilisé dans toutes les compositions où l'on recherche, en particulier, à inhiber l'action de la 3',5'-AMPc phosphodiestérase.

Ainsi, cette utilisation est avantageusement aussi caractérisée en ce que l'extrait précité est un agent inhibiteur de la 3',5'-AMPc phosphodiestérase.

Selon un mode de réalisation avantageux de cette utilisation, celle-ci est caractérisée en ce que l'extrait est destiné au soin de la peau, en particulier pour obtenir une action amincissante, pour retarder ou atténuer les effets du vieillissement de la peau, pour réduire, éliminer ou prévenir les surcharges graisseuses sous-cutanées, notamment la cellulite.

Cet agent cosmétique pourra donc être utilisé pour lutter contre les effets du vieillissement sur la peau, notamment en préservant ou améliorant les qualités biomécaniques de la peau, en particulier en améliorant la fermeté cutanée, l'élasticité de la peau, en retardant l'apparition des rides ou en diminuant leur profondeur.

Cet agent cosmétique pourra encore être utilisé pour obtenir un amincissement de différentes parties du corps, en particulier des hanches.

Cet agent cosmétique est en particulier utilisé pour lutter contre la cellulite.

Ainsi, les compositions cosmétiques de l'invention seront notamment utilisées pour toutes les applications cosmétiques où l'on cherche à inhiber la PDE.

Comme on l'a vu précédemment, l'efficacité des compositions cosmétiques précédemment décrites a pu être corrélée avec une activité enzymatique. La mise en évidence de cette activité a permis d'envisager également l'utilisation des extraits lipidiques définis précédemment pour la préparation de compositions pharmaceutiques, notamment dermatologiques dans lesquelles une telle activité est souhaitée. Les essais réalisés par les inventeurs de la présente invention ont confirmé l'efficacité de ces compositions pharmaceutiques.

Ainsi, l'inhibition de la PDE se traduit par le maintien d'un taux élevé d'AMPc intracellulaire. Ceci conduit à divers effets intéressants pour la peau, en particulier des effets amincissants, anti-vieillissements, anti-inflammatoires et anti-cellulite.

Selon encore un quatrième aspect, la présente invention concerne un procédé de soin cosmétique, caractérisé en ce qu'il comprend l'application topique, en particulier sur la peau, d'une personne en ayant besoin, d'une quantité cosmétiquement efficace, d'un extrait lipidique de l'algue Skeletonema, notamment de l'algue Skeletonema costatum, en particulier d'un extrait lipidique total de ladite algue, tel que défini précédemment éventuellement dans un excipient cosmétiquement acceptable.

D'autres caractéristiques du procédé apparaissent clairement de la description précédente et suivante prise dans son ensemble.

En particulier, selon une variante de réalisation, il s'agit d'un procédé de soin cosmétique amincissant comprenant l'application sur les zones de la peau concernées d'une personne en ayant besoin, d'une quantité efficace de l'extrait lipidique précité de l'algue Skeletonema pour obtenir un effet amincissant.

Selon une autre variante de réalisation, il s'agit d'un soin cosmétique anti-cellulitique comprenant l'application sur les zones de la peau concernées d'une personne en ayant besoin, d'une quantité efficace de l'extrait lipidique précité de l'algue Skeletonema pour obtenir un effet de réduction de la cellulite ou pour en retarder l'apparition ou la progression.

Selon encore une autre variante de réalisation, il s'agit d'un soin cosmétique anti-vieillissement de la peau comprenant l'application sur les zones de la peau concernées d'une personne en ayant besoin, d'une quantité efficace de l'extrait lipidique précité de l'algue Skeletonema pour obtenir un effet anti-vieillissement sur lesdites zones de la peau, notamment pour améliorer la fermeté cutanée, améliorer l'élasticité de la peau, retarder l'apparition des rides ou diminuer leur profondeur.

Ce soin cosmétique peut également consister en un soin destiné à réduire les surcharges graisseuses cutanées et en particulier destiné à lutter contre la cellulite.

Selon encore un autre aspect, l'invention concerne un procédé de soin cosmétique destiné au maintien ou à l'obtention d'un taux élevé d'AMPc intracellulaire au niveau des cellules cutanées, caractérisé en ce qu'il comprend l'application topique sur la peau d'une quantité cosmétiquement efficace d'un extrait lipidique précité de l'algue Skeletonema, notamment de l'algue Skeletonema costatum, en particulier d'un extrait lipidique total de ladite algue, pour maintenir ou obtenir ledit taux élevé d'AMPc intracellulaire.

Dans chacun des aspects précédents, l'algue Skeletonema est de préférence choisie parmi le groupe consistant de l'algue Skeletonema tropicum, Skeletonema menzelii, Skeletonema potamos, Skeletonema subsalsum, Skeletonema pseudocostatum et Skeletonema costatum. Encore de préférence, l'algue Skeletonema est l'algue Skeletonema costatum.

Par ailleurs, dans le cadre d'une composition cosmétique ou dans le cas d'une utilisation cosmétique, l'extrait lipidique de l'algue Skeletonema est un extrait lipidique obtenu avec un solvant quelconque, c'est-à-dire qu'il n'est pas limité aux extraits et au procédé d'extraction décrits et revendiqués. Il est davantage préféré d'utiliser un extrait lipidique de l'algue Skeletonema avec un solvant organique de faible polarité, ayant de préférence un indice p' inférieur à environ 5,4, de préférence compris entre 2 et 4,5, et encore de préférence compris entre 4,2 et 4,4. Les solvants particuliers précédemment cités répondant à ces critères sont évidement utilisables dans chacun des aspects de l'invention.

En outre, le mode de réalisation préféré de l'invention est relatif à l'emploi d'un extrait lipidique de l'algue Skeletonema costatum, et de préférence un extrait lipidique de l'algue Skeletonema costatum comme précédemment défini.

Avantageusement, l'extrait lipidique de l'algue Skeletonema, en particulier d'un extrait lipidique total de ladite algue, est obtenu à partir de l'algue congelée extraite dans l'isopropanol, de préférence au reflux.

Les exemples qui suivent sont donnés à titre purement illustratif de l'invention.

D'autres avantages de l'invention apparaîtront au vu de la description et des exemples qui suivent.

Sauf indication contraire, les proportions données dans les exemples de compositions sont exprimées en pourcentage en poids.

Exemple 1

Préparation d'un extrait lipidique selon l'invention

De préférence, l'ensemble de l'extraction sera réalisé sous atmosphère inerte (saturation en azote) afin d'éviter une dégradation prononcée des molécules actives.

Dans cette préparation, nous utilisons 250 kg de biomasse (Skeletonema costatum).

Les algues, qui ont été congelées à -20°C, sont plongées dans l'IPA (isopropanol) à reflux à 80-83 °C, et ce sous agitation. Le choc thermique permet de faciliter la décantation de la silice (issue du squelette des cellules des algues).

La quantité de solvant utilisée est de 10 litres d'IPA pour 1 litre d'eau contenue dans la biomasse. Dans cette préparation, pour un pourcentage de matière sèche de 30 %, les 250 kg de biomasse représentent une quantité de matière sèche de 75 kg et 175 kg d'eau. La quantité d'IPA engagé est ici de 1925 kg.

L'ensemble (biomasse + IPA) est porté à reflux pendant une demi-heure sous agitation (et donc à environ 80°C) avant d'être refroidi vers 50°C.

Après le refroidissement de la masse réactionnelle vers 50°C, l'extrait est transféré dans un filtre de type GUEDU afin de réaliser la séparation biomasse épuisée / extrait lipidique sous IPA.

L'extrait lipidique est concentré dans un réacteur batch (Facteur de concentration = 71,5).

Le rendement par rapport au poids sec de cette première étape est de 28 % en huile brute.

Pour commencer la seconde étape, l'extrait lipidique est repris dans de l'IPA à froid à raison de 10 kg de solvant pour 1 kg d'huile. L'agitation est prolongée pendant 20 minutes. Le jus est ensuite filtré (ceci permet d'éliminer la boue collante résiduelle).

Le traitement de décoloration et de désodorisation est réalisé en deux batchs dans un réacteur schott de 80 litres et dure 30 minutes à température ambiante. La quantité de zéolithe (ABSENT 2000, fournisseur UOP) ajoutée est de 0,94 kg et celle de charbon actif (CXV, fournisseur CECA) est de 1,6 kg. Le rapport charbon sur zéolithe est de 1,7.

La zéolithe et le charbon sont ensuite éliminés par filtration sur papier.

Les rendements par rapport au poids sec de cette seconde étape est de 37 %.

Ainsi, le rendement global en huile pour l'ensemble du procédé est de 10 % par rapport au poids sec de biomasse.

Des antioxydants (du DL $\alpha$-tocophérol à 0,05% final en poids et du palmitate d'ascorbyle à 0,05 % final en poids) sont incorporés par une solution mère dans de l'IPA.

Le filtrat et les antioxydants sont ensuite concentrés en batch jusqu'à l'obtention d'une huile de couleur brune.

Le conditionnement de cet extrait lipidique est réalisé sous gaz inerte tel que de l'azote.

Exemple 2

Mise en évidence de l'activité inhibitrice des extraits lipidiques sur la PDE

**2.1- Principe du test d'activité inhibitrice :**

Le principe du test est basé sur l'hydrolyse de la 3',5'-adénosine monophosphate cyclique (AMPc) en adénosine monophosphate (AMP). La formation d'AMP est mesurée par analyse HPLC (Chromatographie Liquide Haute Performances).

Les solutions des réactifs sont réalisées dans du tampon Tris-HCL : 0,05M à pH=7,5.

Les extraits lipidiques à tester sont à 0,1% dans du DMSO.

On réalise la même expérience avec un produit actif de référence, la théophilline (H. Kather, A. Scheurer, Effects of different phosphodiesterase inhibitors on the antilipolytic action of insulin in human adipocytes : Horm. Metabol. Res. 19 (1987) 379-381). De même, B.B. Fredholm, E. Lindger, Acta pharmacol. et toxicol., 54 (1984), p. 64-71, "The effect of Alkylxanthines and other Phosphodiesterase Inhibitors on Adenosine receptor mediated decrease in lipolysis and cyclic AMP accumulation in rat fat cells". Ces tests sont des tests de référence pour l'homme de l'art.

La théophilline testée (SIGMA / Réf. T1633) est à 0,2 % dans du DMSO.

La composition des milieux réactionnels est décrite ci-après. On mélange la solution d'AMPc avec le solvant qui contient ou non le produit à tester et le tampon. A ce milieu on ajoute extemporanément la PDE qui est dans du tampon Tris-HCL.

On mesure la quantité d'AMP formée après 5 minutes de réaction, en calculant la surface d'intégration du pic de l'AMP sur le chromatogramme fourni par l'appareil HPLC (KONTRON S.A.).

La composition des milieux réactionnels est indiquée ci-dessous :

|  | Echantillon Témoin | Echantillon avec produit testé |
|---|---|---|
| Substrat (AMPc (WALDHOF / Allemagne) : 0,25% dans le tampon) | 80μl | 80μl |
| Solvant DMSO (Dimethyl sulfoxide) | 80μl | 0 |
| Produit testé (à 0,1% dans du DMSO) | 0 | 80μl |
| Tampon Tris-HCL : 0,05M à pH=7,5 | 480μl | 480μl |
|  |  |  |
| Ajouter extemporanément : |  |  |
|  |  |  |
| Enzyme (PDE (SIGMA / Réf. : P800) : 0,5 U/ml dans le tampon) | 160μl | 160μl |

2.2- Le mode de calcul des résultats est le suivant :

On mesure la quantité d'AMP due à l'action de la PDE, après 5 minutes de réaction.

On obtient une activité " A " du produit testé qui est exprimé sous la forme d'un pourcentage d'inhibition de la PDE définie ci-dessous :

$$A = (\frac{S_t\text{-}S_p}{S_t}) \times 100$$

Avec :

$S_t$ qui est la surface d'intégration de l'AMP pour l'échantillon témoin
$S_p$ qui est la surface d'intégration de l'AMP pour l'échantillon avec produit testé.

2.3- Les résultats

Tableau I

| Inhibition de la PDE | | | |
|---|---|---|---|
|  | Surface d'intégration de l'AMP | A (%) | Ecart type |
| DMSO | 40,80 | 0 | 0,6 |
| théophilline | 26,52 | 35 | 2,5 |
| Extrait lipidique sec de Skeletonema costatum | 23,26 | 43 | 4 |

2.4- Commentaires des résultats

Les résultats obtenus démontrent l'existence d'une importante activité inhibitrice de l'extrait lipidique selon l'invention sur la PDE.

Cette activité inhibitrice de l'extrait selon l'invention est au moins aussi importante que celle, bien connue, de la théophilline. Elle est même peut-être supérieure à celle de la théophilline.

Grâce à l'inhibition de la PDE, d'une manière très significative, l'invention permet de maintenir un taux intracellulaire élevé d'AMPc, ce qui a pour effet notamment d'activer les protéines kinases A, et, par ce processus, permet en particulier de favoriser la dégradation des lipides. L'invention permet donc d'avoir une action amincissante.

D'autre part, étant donné que l'AMPc intervient pour contrer certains processus inflammatoires, l'extrait de la plante Skeletonema selon l'invention a une activité anti-inflammatoire.

La combinaison des actions de dégradation des lipides et de limitation des processus inflammatoires permet de lutter contre la cellulite.

Grâce également au fait que la PDE augmente avec l'âge, l'activité inhibitrice de la PDE obtenue avec l'extrait selon l'invention permet de retarder l'apparition des signes du vieillissement. Les présents essais démontrent bien que l'extrait de Skeletonema selon la présente invention peut être utilisé en cosmétique ou pharmacie pour obtenir l'ensemble des effets précédemment décrits.

Exemple 3

Crème peaux sensibles.

| | |
|---|---|
| Huile minérale fluide | 7,50 g |
| Octanoate d'octyl | 5,00 g |
| Octanoate de cétostéaryl | 5,00 g |
| Méthyl glucose sesquistéarate | 3,00 g |
| Cire d'abeille | 3,00 g |
| Glycérine | 3,00 g |
| Gomme Xanthane | 0,50 g |
| Parfum | 0,30 g |
| Phénoxyéthanol | 0,20 g |
| Extrait lipidique sec de Skeletonema costatum | 0,05 g |
| Colorant | qs. g |
| Eau | qsp 100,00 g |

Exemple 4

Lotion adoucissante

| | |
|---|---|
| Eau florale Hannarelis | 5,00 g |
| Méthyl glucose POE 20 | 2,00 g |
| Glycérine | 2,00 g |
| Polysorbate 20 | 1,50 g |
| Parahydroxybenzoate de méthyl | 0,15 g |
| EDTA tétrasodique | 0,10 g |
| Parfum | 0,10 g |
| Extrait lipidique sec de Skeletonema costatum | 0,05 g |
| Colorant | qs. g |
| Acide citrique / Citrate de sodium | qsp (pH = 6,50) g |
| Eau | qsp 100 g |

Exemple 5

Gel hydroalcoolique amincissant.

| Alcool dénaturé (éthanol) | 30,00 g |
|---|---|
| Glycérine | 2,00 g |
| Carbomer | 0,50 g |
| Triéthanolamine | 0,50 g |
| Extrait lipidique sec de Skeletonema costatum | 0,10 g |
| Eau | qsp 100,00 g |

**Revendications**

**1.** Utilisation d'un extrait lipidique de l'algue Skeletonema, obtenu par extraction dans un solvant organique, pour la fabrication d'une composition cosmétique, pour réaliser un soin cosmétique choisi parmi une action amincissante, la réduction, l'élimination et la prévention des surcharges graisseuses sous-cutanées, notamment la cellulite.

**2.** Utilisation d'un extrait lipidique de l'algue Skeletonema, obtenu par extraction dans un solvant organique, pour la fabrication d'une composition cosmétique destinée aux soins de la peau pour inhiber la 3',5'-AMPc phosphodiestérase.

**3.** Utilisation selon la revendication 1 ou 2, **caractérisée en ce que** l'algue Skeletonema est choisie dans le groupe consistant de S. tropicum, S. menzelii, S. potamos, S. subsalsum, S. pseudocostatum et S. costatum.

**4.** Utilisation selon l'une des revendications 1 à 3, **caractérisée en ce qu'**il s'agit d'un extrait lipidique obtenu par extraction de l'algue Skeletonema dans un solvant organique, présentant un indice de polarité p' inférieur à environ 5,4, de préférence compris entre 2 et 4,5, et encore de préférence p' étant compris entre 4,2 et 4,4, et acceptable dans l'industrie cosmétique ou pharmaceutique.

**5.** Utilisation selon l'une des revendications 1 à 4, **caractérisée en ce que** l'extrait précité est obtenu par extraction de l'algue avec un solvant organique choisi parmi le groupe consistant de l'isopropanol, l'acétate d'éthyle, le dichlorométhane et le chloroforme.

**6.** Utilisation selon l'une revendications 1 à 5, **caractérisée en ce que** l'extrait précité est obtenu par extraction de l'algue avec de l'isopropanol.

**7.** Utilisation selon l'une des revendications précédentes, **caractérisée en ce que** l'extraction est réalisée au reflux.

**8.** Utilisation selon l'une des revendications précédentes, **caractérisée en ce que** l'algue est congelée avant d'être extraite avec le solvant, de préférence la congélation étant réalisée à une température située entre -40°C et -20°C environ et pendant une durée comprise de préférence entre 1 et 7 jours environ.

**9.** Utilisation selon la revendication 8, **caractérisée en ce que** l'algue congelée est plongée directement dans le solvant porté à température de reflux.

**10.** Utilisation selon l'une des revendications 1 à 9, **caractérisée en ce que**, avant toute opération d'extraction, on réalise une macération de l'algue dans le solvant à température ambiante, de préférence pendant une durée comprise entre 5 minutes et 80 minutes environ, et de préférence encore, pendant une durée comprise entre 20 minutes et 40 minutes environ.

**11.** Utilisation selon l'une des revendications précédentes, **caractérisée en ce que** la quantité de solvant utilisée est comprise entre 0,1 litre et 20 litres environ de solvant, de préférence comprise entre 2 litres et 10 litres environ de solvant, pour 100 g d'algue, exprimé en poids sec d'algue.

12. Utilisation selon l'une des revendications précédentes, **caractérisée en ce que** l'extraction est réalisée sous atmosphère inerte, de préférence sous atmosphère saturée en azote.

13. Utilisation selon l'une des revendications 1 à 12, **caractérisée en ce que** l'extrait lipidique de l'algue Skeletonema précité est incorporé dans une composition cosmétique, qui comprend de 0,001 % à 10 % environ, et en particulier de 0,02 % à 1 % environ en poids sec dudit extrait lipidique par rapport au poids total de la composition finale.

14. Utilisation selon l'une des revendications 1 à 13, **caractérisée en ce que** l'extrait lipidique précité est utilisé avec au moins un agent antioxydant, en particulier le DL $\alpha$-tocophérol, ou l'un de ses sels ou esters tel que le tocophérolphosphate, et/ou un ascorbate, en particulier le palmitate d'ascorbyle, de préférence à une concentration comprise entre 0,001 % et 5 % environ en poids, par rapport au poids total dudit extrait.

15. Procédé de soin cosmétique, **caractérisé en ce qu'**il comprend l'application topique, sur la peau d'une personne en ayant besoin, d'une quantité cosmétiquement efficace d'un extrait lipidique de l'algue Skeletonema, en particulier d'un extrait lipidique total de ladite algue, tel que défini à l'une quelconque des revendications 1 à 12, éventuellement dans un excipient cosmétiquement acceptable ; pour réaliser un soin choisi parmi une action amincissante, la réduction, l'élimination et la prévention des surcharges graisseuses sous-cutanées, notamment la cellulite.

16. Procédé selon la revendication 15, **caractérisé en ce qu'**il s'agit d'un soin cosmétique amincissant comprenant l'application sur les zones de la peau concernée d'une personne en ayant besoin, d'une quantité efficace de l'extrait lipidique précité de l'algue Skeletonema pour obtenir un effet amincissant.

17. Procédé selon la revendication 15 ou 16, **caractérisé en ce qu'**il s'agit d'un soin cosmétique anti-cellulitique comprenant l'application sur les zones de la peau concernée d'une personne en ayant besoin, d'une quantité efficace de l'extrait lipidique précité de l'algue Skeletonema pour obtenir un effet de réduction de la cellulite ou pour en retarder l'apparition ou la progression.

18. Procédé de soin cosmétique destiné au maintien ou à l'obtention d'un taux élevé d'AMPc intracellulaire au niveau des cellules cutanées, **caractérisé en ce qu'**il comprend l'application topique sur la peau d'une quantité cosmétiquement efficace d'un extrait lipidique de l'algue Skeletonema, notamment de l'algue Skeletonema costatum, en particulier d'un extrait lipidique total de ladite algue, tel que défini à l'une quelconque des revendications 1 à 12, pour maintenir ou obtenir ledit taux élevé d'AMPc intracellulaire.

19. Procédé selon l'une des revendications 15 à 18, **caractérisé en ce que** l'algue Skeletonema précitée est choisie dans le groupe consistant de S. tropicum, S. menzelii, S. potamos, S. subsalsum, S. pseudocostatum et S. costatum.

20. Procédé selon l'une des revendications 15 à 19, **caractérisé en ce qu'**il s'agit d'un extrait lipidique de l'algue Skeletonema, notamment de l'algue Skeletonema costatum, en particulier d'un extrait lipidique total de ladite algue, obtenu à partir de l'algue congelée extraite dans l'isopropanol, de préférence au reflux.

21. Procédé selon l'une des revendications 15 à 20, **caractérisé en ce que** l'extrait lipidique de l'algue Skeletonema est incorporé dans une composition cosmétique comprenant de 0,001 % à 10 % environ, et en particulier de 0,02 % à 1 % environ en poids sec dudit extrait lipidique par rapport au poids total de la composition finale.

22. Procédé selon l'une des revendications 15 à 21, **caractérisé en ce que** l'extrait lipidique précité est utilisé avec au moins un agent antioxydant, en particulier le DL $\alpha$-tocophérol, ou l'un de ses sels ou esters tel que le tocophérolphosphate, et/ou un ascorbate, en particulier le palmitate d'ascorbyle, de préférence à une concentration comprise entre 0,001 % et 5 % environ en poids, par rapport au poids total dudit extrait.

**Patentansprüche**

1. Verwendung eines Lipidextrakts der Alge Skeletonema, der durch die Extraktion mit einem organischen Lösungsmittel erhalten wurde, zur Herstellung einer kosmetischen Zusammensetzung, um eine kosmetische Behandlung durchzuführen, die aus einer schlankmachenden Wirkung, der Reduzierung, Eliminierung und Verhinderung von subkutanen Fettüberschüssen, insbesondere der Cellulitis, ausgewählt wird.

2. Verwendung eines Lipidextrakts der Alge Skeletonema, der durch die Extraktion mit einem organischen Lösungsmittel erhalten wurde zur Herstellung einer kosmetischen Zusammensetzung, die auf die Hautpflege ausgerichtet ist, um die 3',5'-cAMP-Phosphodiesterase zu hemmen.

3. Verwendung gemäß Anspruch 1 oder 2, **dadurch gekennzeichnet, dass** die Alge Skeletonema aus der Gruppe ausgewählt wird, die aus S. tropicum, S. menzelii, S. potamos, S. subsalsum, S. pseudocostatum und S. costatum besteht.

4. Verwendung gemäß einem der Ansprüche 1 bis 3, **dadurch gekennzeichnet, dass** es sich um einen Lipidextrakt handelt, der durch die Extraktion der Alge Skeletonema mit einem organischen Lösungsmittel erhalten wird, das einen Polaritätsindex p' unterhalb ungefähr 5,4, bevorzugt zwischen 2 und 4,5 aufweist, und noch bevorzugter liegt p' zwischen 4,2 und 4,4, und ist in der kosmetischen oder pharmazeutischen Industrie akzeptabel.

5. Verwendung gemäß einem der Ansprüche 1 bis 3, **dadurch gekennzeichnet, dass** der oben erwähnte Extrakt durch die Extraktion der Alge mit einem organischen Lösungsmittel erhalten wird, das aus der Gruppe ausgewählt wird, die aus Isopropanol, Ethylacetat, Dichlormethan und Chloroform besteht.

6. Verwendung gemäß einem der Ansprüche 1 bis 5, **dadurch gekennzeichnet, dass** der oben erwähnte Extrakt durch die Extraktion der Alge mit Isopropanol erhalten wird.

7. Verwendung gemäß einem der zuvor vorhergehenden Ansprüche, **dadurch gekennzeichnet, dass** die Extraktion mittels eines Rückflusses durchgeführt wird.

8. Verwendung gemäß einem der vorhergehenden Ansprüche, **dadurch gekennzeichnet, dass** die Alge eingefroren wird, bevor sie mit dem Lösungsmittel extrahiert wird, wobei das Einfrieren bevorzugt bei einer Temperatur zwischen ungefähr -40°C und -20°C und vorzugsweise für eine Dauer von ungefähr zwischen 1 und 7 Tagen durchgeführt wird.

9. Verwendung gemäß Anspruch 8, **dadurch gekennzeichnet, dass** die eingefrorene Alge direkt in das Lösungsmittel eingetaucht wird, das auf die Rückflusstemperatur gebracht wurde.

10. Verwendung gemäß einem der Ansprüche 1 bis 9, **dadurch gekennzeichnet, dass** vor jedem Extraktionsvorgang bei Raumtemperatur eine Mazerierung der Alge in dem Lösungsmittel durchgeführt wird, bevorzugt für eine Dauer zwischen ungefähr 5 Minuten und 80 Minuten, und noch bevorzugter für eine Dauer zwischen ungefähr 20 Minuten und 40 Minuten.

11. Verwendung gemäß einem der zuvor erwähnten Ansprüche, **dadurch gekennzeichnet, dass** die Menge des verwendeten Lösungsmittels zwischen ungefähr 0,1 Liter und 20 Litern des Lösungsmittels beträgt, bevorzugt zwischen 2 Litern und ungefähr 10 Litern des Lösungsmittels für 100 g an Algen, bezogen auf das Trockengewicht der Algen.

12. Verwendung gemäß einem der vorhergehenden Ansprüche, **dadurch gekennzeichnet, dass** die Extraktion in einer inerten Atmosphäre durchgeführt wird, bevorzugt in einer Stickstoff-gesättigten Atmosphäre.

13. Verwendung gemäß einem der Ansprüche 1 bis 12, **dadurch gekennzeichnet, dass** der oben erwähnte Lipidextrakt der Alge Skeletonema in einer kosmetischen Zusammensetzung enthalten ist, die ungefähr 0,001% bis 10%, und insbesondere ungefähr 0,02% bis 1% an Trockengewicht des Lipidextraktes umfasst, bezogen auf das Gesamtgewicht der endgültigen Zusammensetzung.

14. Verwendung gemäß einem der Ansprüche 1 bis 13, **dadurch gekennzeichnet, dass** der oben erwähnte Lipidextrakt mit mindestens einem Antioxidationsmittel verwendet wird, insbesondere dem DLα-Tocopherol oder einem seiner Salze oder Ester, wie dem Tocopherolphosphat, und/oder einem Ascorbat, insbesondere dem Ascorbylpalmitat, bevorzugt in einer Konzentration zwischen ungefähr 0,001 Gew.% und 5 Gew.% bezogen auf das Gesamtgewicht des Extrakts.

**15.** Verfahren zur kosmetischen Behandlung, **dadurch gekennzeichnet, dass** es die topische Anwendung einer kosmetisch wirksamen Menge eines Lipidextrakts der Alge Skeletonema, insbesondere eines Gesamtlipidextrakts dieser Alge, wie in einem der Ansprüche 1 bis 12 definiert, gegebenenfalls in einem kosmetisch akzeptablen Träger auf der Haut einer Person umfasst, die dessen bedarf; zum Durchführen einer Behandlung, die aus einer schlankmachenden Wirkung, der Reduzierung, Eliminierung und Verhinderung von subkutanen Fettüberschüssen, insbesondere der Cellulitis, ausgewählt ist.

**16.** Verfahren gemäß Anspruch 15, **dadurch gekennzeichnet, dass** es sich um eine schlankmachende kosmetische Behandlung handelt, die die Anwendung einer wirksamen Menge des oben erwähnten Lipidextrakts der Alge Skeletonema auf betroffenen Hautbereichen einer Person umfasst, die dessen bedarf, um eine schlankmachende Wirkung zu erhalten.

**17.** Verfahren gemäß einem der Ansprüche 15 oder 16, **dadurch gekennzeichnet, dass** es sich um eine kosmetische Anti-Cellulitis-Behandlung handelt, die das Auftragen einer wirksamen Menge des oben erwähnten Lipidextrakts der Alge Skeletonema auf den betroffenen Hautbereichen einer Person umfasst, die dessen bedarf, um eine Cellulitisreduzierende Wirkung zu erhalten oder um deren Auftreten oder Voranschreiten zu verzögern.

**18.** Verfahren zur kosmetischen Behandlung, das auf die Bewahrung oder das Erreichen einer erhöhten Menge intrazellulären cAMPs im Bereich der Hautzellen ausgerichtet ist, **dadurch gekennzeichnet, dass** es die topische Anwendung einer kosmetisch wirksamen Menge eines Lipidextrakts der Alge Skeletonema umfasst, insbesondere der Alge Skeletonema costatum, insbesondere eines Gesamtlipidextrakts dieser Alge, wie in einem der Ansprüche 1 bis 12 definiert, um die erhöhte intrazelluläre cAMP-Menge zu bewahren oder zu erhalten.

**19.** Verfahren gemäß einem der Ansprüche 15 bis 18, **dadurch gekennzeichnet, dass** die oben erwähnte Alge aus der Gruppe ausgewählt ist, die aus S. tropicum, S. menzelii, S. potamos, S. subsalsum, S. pseudocostatum und S. costatum besteht.

**20.** Verfahren gemäß einem der Ansprüche 15 bis 19, **dadurch gekennzeichnet, dass** es sich um einen Lipidextrakt der Alge Skeletonema, insbesondere der Alge Skeletonema costatum, insbesondere eines Gesamtlipidextrakts dieser Alge handelt, der aus einer eingefrorenen Alge, die mit Isopropanol extrahiert wurde, vorzugsweise durch Rückfluss erhalten wurde.

**21.** Verfahren gemäß einem der Ansprüche 15 bis 20, **dadurch gekennzeichnet, dass** der Lipidextrakt der Alge Skeletonema in einer kosmetischen Zusammensetzung enthalten ist, die ungefähr 0,001% bis 10%, und insbesondere ungefähr 0,02% bis 1% an Trockengewicht des Lipidextrakts umfasst, bezogen auf das Gesamtgewicht der endgültigen Zusammensetzung.

**22.** Verfahren gemäß einem der Ansprüche 15 bis 21, **dadurch gekennzeichnet, dass** der oben erwähnte Lipidextrakt mit mindestens einem Anti-Oxidationsmittel verwendet wird, insbesondere dem DL$\alpha$-Tocopherol, einem seiner Salze oder Ester, wie dem Tocopherolphosphat, und/oder einem Ascorbat, insbesondere dem Ascorbylpalmitat, bevorzugt in einer Konzentration zwischen ungefähr 0,001 Gew.% und 5 Gew.%, bezogen auf das Gesamtgewicht des Extrakts.

**Claims**

**1.** Use of a lipid extract of the algae Skeletonema, obtained by extraction in an organic solvent, for the manufacture of a cosmetic composition, to perform a cosmetic care selected among a slimming action, reduction, elimination and prevention of excessive sub-cutaneous fat deposits, notably cellulite.

**2.** Use of a lipid extract of the algae Skeletonema, obtained by extraction in an organic solvent, for the manufacture of a cosmetic composition aimed to the skin care to inhibit 3', 5'-AMPc phosphodiesterase.

**3.** Use according to claim 1 or 2, **characterized in that** the algae is selected from the group consisting of S. tropicum, S. menzelii, S. potamos, S. subsalsum, S. pseudocostatum and S. costatum.

**4.** Use according to one of claims 1 to 3, **characterized in that** it is a lipid extract obtained by extracting the algae Skeletonema in an organic solvent which has a polarity index p' of less than about 5.4, preferably of between 2 and 4.5 and particularly preferably of between 4.2 and 4.4, and which is acceptable in the cosmetic or pharmaceutical industry.

5. Use according to one of claims 1 to 4, **characterized in that** the extract is obtained by extracting the algae with an organic solvent selected from the group consisting of isopropanol, ethyl acetate, dichloromethane and chloroform.

6. Use according to any one of claims 1 to 5, **characterized in that** the extract is obtained by extracting the algae with isopropanol.

7. Use according to one of the preceding claims, **characterized in that** the extraction is performed under reflux.

8. Use according to one of the preceding claims, **characterized in that** the algae is frozen before being extracted with the solvent, the freezing preferably being effected at a temperature of between about -40°C and -20°C and for a period preferably of between about 1 and 7 days.

9. Use according to claim 8, **characterized in that** the frozen algae is immersed directly in the solvent heated to the reflux temperature.

10. Use according to one of claims 1 to 9, **characterized in that**, before any extraction operation, the algae is macerated in the solvent at room temperature, preferably for a period of between about 5 minutes and 80 minutes and particularly preferably for a period of between about 20 minutes and 40 minutes.

11. Use according to one of the preceding claims, **characterized in that** the amount of solvent used is ranging between about 0.1 liter and 20 liters, preferably between about 2 liters and 10 liters, per 100 g of algae, expressed by dry weight of algae.

12. Use according to one of the preceding claims, **characterized in that** the extraction is performed under an inert atmosphere, preferably under a nitrogen-saturated atmosphere.

13. Use according to one of claims 1 to 12, **characterized in that** the lipid extract of the algae Skeletonema is incorporated in a cosmetic composition which comprises from about 0.001% to 10%, particularly from about 0.02% to 1%, by dry weight of said lipid extract, based on the total weight of the final composition.

14. Use according to one of claims 1 to 13, **characterized in that** the lipidic extract is used with at least one antioxidant, particularly D,L-$\alpha$-tocopherol or one of its salts or esters such as tocopherol phosphate, and/or an ascorbate, particularly ascorbyl palmitate, preferably at a concentration of between about 0.001% and 5% by weight, based on the total weight of said extract.

15. Method of cosmetic care, **characterized in that** it comprises the topical application, to the skin of a person in need thereof, of a cosmetically effective amount of a lipid extract of the algae Skeletonema, in particular a total lipid extract of said algae, as defined in any one of claims 1 to 12, optionally in a cosmetically acceptable excipient; to perform a care selected from a slimming action, reduction, elimination and prevention of excessive sub-cutaneous fat deposits, notably cellulite.

16. Method according to claim 15, **characterized in that** the cosmetic care is a cosmetic slimming treatment comprising the application, to the appropriate skin areas of a person in need thereof, of an effective amount of the lipid extract of the algae Skeletonema for obtaining a slimming effect.

17. Method according to claim 15 or 16, **characterized in that** the cosmetic care is a cosmetic anti-cellulite treatment comprising the application, to the appropriate skin areas of a person in need thereof, of an effective amount of the lipid extract of the algae Skeletonema for obtaining a cellulite-reducing effect or for delaying the appearance or development of cellulite.

18. Method of cosmetic care for maintaining or obtaining a high level of intracellular cAMP in the skin cells, **characterized in that** it comprises the topical application, to the skin, of a cosmetically effective amount of a lipid extract of the algae Skeletonema, especially the algae Skeletonema costatum, and in particular a total lipid extract of said algae, particularly as defined in any one of claims 1 to 12, for maintaining or obtaining said high level of intracellular cAMP.

19. Method according to one of claims 15 to 18, **characterized in that** the algae is selected from the group consisting of S. tropicum, S. menzelii, S. potamos, S. subsalsum, S. pseudocostatum and S. costatum.

20. Method according to one of claims 15 to 19, **characterized in that** the extract is a lipid extract of the algae Skeletonema, especially the algae Skeletonema costatum, and in particular a total lipid extract of said algae, obtained from the frozen algae extracted in isopropanol, preferably under reflux.

21. Method according to one of claims 15 to 20, **characterized in that** said lipid extract of the algae Skeletonema is incorporated in a cosmetic composition comprising from about 0.001 % to 10 %, and in particular from about 0.02 % to 1 % in dry weight of said lipid extract with respect to the total weight of the final composition.

22. Method according to one of claims 15 to 21, **characterized in that** said lipid extract is used with at least one anti-oxidant particularly D,L-$\alpha$-tocopherol or one of its salts or esters such as tocopherol phosphate, and/or an ascorbate, particularly ascorbyl palmitate, preferably at a concentration of between about 0.001% and 5% by weight, based on the total weight of said extract.